# EUROPÄISCHE PATENTSCHRIFT

(11) **EP 0 848 711 B1**
(45) Veröffentlichungstag und Bekanntmachung des Hinweises auf die Patenterteilung: **24.01.2001**
(21) Anmeldenummer: 96930978.0
(22) Anmeldetag: 29.08.1996
(51) Int. Cl.: C07D 301/00, C07D 303/04

(54) **VERFAHREN ZUR SELEKTIVEN HYDRIERUNG VON VINYLOXIRAN ZU BUTYLENOXID**
METHOD FOR THE SELECTIVE HYDROGENATION OF VINYL OXIRANE TO BUTYLENE OXIDE
PROCEDE D'HYDROGENATION SELECTIVE D'OXYRANE VINYLIQUE POUR FORMER DE L'OXYDE DE BUTYLENE

(30) Priorität: 05.09.1995 DE 19532645
(43) Veröffentlichungstag der Anmeldung: 24.06.1998
(73) Patentinhaber: BASF AKTIENGESELLSCHAFT, 67056 Ludwigshafen (DE)
(72) Erfinder: SIGWART, Christoph, D-69198 Schriesheim (DE); HARTH, Klaus, D-67317 Altleiningen (DE); FISCHER, Rolf, D-69121 Heidelberg (DE)
(86) Internationale Anmeldenummer: EP9603799
(87) Internationale Veröffentlichungsnummer: WO9709321

(56) Entgegenhaltungen:
- EP-A- 0 576 944
- DE-A- 4 407 486
- US-A- 4 536 482
- US-A- 5 077 418

## Beschreibung

Die vorliegende Erfindung betrifft ein verbessertes Verfahren zur Herstellung von 1,2-Butylenoxid durch katalytische Hydrierung von Vinyloxiran an Heterogenkatalysatoren.

Die heterogen-katalytische Hydrierung von Vinyloxiran ist bekannt.

Nach der US-A 2,561,984 entsteht bei der Hydrierung von Vinyloxiran in Ethanol an einem Palladium-Aktivkohle-Katalysator bei 25°C/2 bar nach einer Reaktionszeit von 3 h als Hauptprodukt n-Butyraldehyd. Mit Raney-Nickel als Katalysator wird dagegen bei 25°C und 2 bar nach einer Reaktionszeit von 1,5 h hauptsächlich n-Butanol gebildet. Über die Bildung von Butylenoxid wird nicht berichtet.

In einer Arbeit von Aizikovich et al. (J. Gen. Chem. USSR, 28 (1958) 3076) wird die katalytische Hydrierung von Vinyloxiran in Methanol bzw. Ethanol an Platin-, Palladium- und Raney-Nickel-Katalysatoren beschrieben. Mit einem Palladium-Trägerkatalysator (1,8 Gew.-% Palladium auf Calciumcarbonat) wird bei 15°C/1 bar hauptsächlich n-Butanol gebildet. Als wichtigste Zwischenverbindung der Hydrierung wird in dieser Schrift Crotylalkohol angesehen, obwohl auch die Bildung von n-Butyraldehyd beobachtet wird. Zur Bildung von 1,2-Butylenoxid gibt es auch in dieser Arbeit keine Hinweise.

In den Schriften US-A 5,077,418 und US-A 5,117,013 wird berichtet, daß bei der Hydrierung von Vinyloxiran-Lösungen an Palladium-haltigen Katalysatoren als Hauptprodukt n-Butyraldehyd gebildet wird. So erhält man bei der Hydrierung von Vinlyoxiran mit Tetrahydrofuran als Lösungsmittel an einem Palladium-Aktivkohle-Trägerkatalysator (5 Gew.-% Palladium auf Aktivkohle) bei einer Temperatur von 50 bis 55°C und einem Druck von 3,5 bar nach einer Reaktionszeit von 3 h einen Hydrieraustrag, der 55 % n-Butyraldehyd, nur 27 % 1,2-Butylenoxid und 9 % n-Butanol enthält.

Wird die Hydrierung an Palladium-haltigen Aluminiumoxid-Trägerkatalysatoren (5 % Pd/Al₂O₃) durchgeführt, so werden bei einer Temperatur von 25 bis 55°C und einem Druck von 3,5 bar nach einer Reaktionszeit von 6 h bzw. bei einer Temperatur von 100°C und einem Druck von 20,7 bar nach einer Reaktionszeit von 4 h nur Spuren von 1,2-Butylenoxid gebildet. Bei quantitativem Umsatz entsteht als Hauptprodukt n-Butyraldehyd mit einer Selektivität von 87 % bzw. 78 %.

Außerdem wird die Hydrierung von Vinyloxiran an Raney-Nickel als Hydrierkatalysator bei 50°C und 3,5 bar beschrieben, wobei als Hauptprodukt n-Butanol zu 58 % gebildet wird. Die 1,2-Butylenoxidausbeute ist mit 41 % niedrig. Bei der Hydrierung von Vinyloxiran an einem Platin-haltigen Trägerkatalysator (1 Gew.-% Pt/Al₂O₃) bei 100°C und 20,7 bar Wasserstoffdruck werden nach einer Reaktionszeit von 4,6 h bei vollständigem Umsatz nur 40 % 1,2-Butylenoxid neben 23 % n-Butanol, 24 % an verschiedenen Butenolen, 5 % Crotonaldehyd und 3 % n-Butyraldehyd gefunden. Andere Platin-haltige Katalysatoren liefern noch geringere 1,2-Butylenoxid-Ausbeuten.

Die US-A 5,077,418 und US-A 5,117,013 lehren weiterhin, daß hohe l,2-Butylenoxid-Ausbeuten nur mit Rhodium-haltigen Katalysatoren erhalten werden. Mit verschiedenen Rhodium-haltigen Trägerkatalysatoren (5 Gew.-% Rhodium auf Aktivkohle, 5 Gew.-% Rhodium auf Aluminiumoxid), die allerdings einen hohen Gehalt an dem teuren Edelmetall Rhodium haben, bzw. mit Rhodiumoxidhydrat (Rh₂O₃ · xH₂O) werden bei der Hydrierung von Vinyloxiran-Lösungen 1,2-Butylenoxid-Gehalte von 60-93 % erhalten. Nachteilig an diesem Verfahren ist die geringe Raum-Zeit-Ausbeute, bezogen auf die eingesetzte Rhodiummenge. So beträgt die Raum-Zeit-Ausbeute in Beispiel 2 der US-A 5,117,013 nur 119 kg 1,2-Butylenoxid/kg Rh*h.

In Neftekhimiya 33 (1993) 131 wird die Hydrierung von Vinyloxiran an Nickel-, Palladium- und Kupfer-haltigen Katalysatoren beschrieben. Mit Raney-Nickel bzw. Nickel auf Kieselgur als Katalysator verläuft die Hydrierung hauptsächlich unter Spaltung des Epoxid-Rings, die zur überwiegenden Bildung von 1-Butenolen und n-Butanol führt. Die Ausbeuten an Butylenoxid sind gering. Beispielsweise wird an Raney-Nickel mit Methanol als Lösungsmittel bei 40°C/60 bar Wasserstoffdruck nach 20 min Reaktionszeit bei 94 % Umsatz ein Reaktionsaustrag erhalten, der bezogen auf umgesetztes Vinyloxiran 89 % Butenole, 8 % n-Butanol und nur 2 % 1,2-Butylenoxid enthält. Auch mit frisch hergestelltem Raney-Nikkel (20 Gew.-%) liefert die Hydrierung von Vinyloxiran in Methanol bei 20°C/60 bar H2 nach 3 min Reaktionszeit bei einem Umsatz von 94 % neben 79 % n-Butanol und 6 % Butenol nur 9 % Butylenoxid. Bei einem Hydrierversuche in Methanol bei 20°C/60 bar Wasserstoffdruck an einem mit Isopropanol, Nicotinsäure, Pyridin und Morpholin vorbehandelten Raney-Nickel-Katalysator wird bei 89 % Umsatz mit 37 % die höchste, mit einem Nickel-haltigen Katalysator erzielbare Butylenoxid-Selektivität erreicht. Dabei fallen Butenole bzw. n-Butanol in einer Selektivität von 56 % bzw. 9 % an.

Mit Palladium-haltigen Katalysatoren werden bei der Hydrierung von Vinyloxiran im Vergleich zu den Versuchen mit Nickel-haltigen Katalysatoren höhere Butylenoxid-Selektivitäten erzielt. Beispielsweise wird mit einem Palladium/Aktivkohle-Katalysator ohne Einsatz eines Lösungsmittels bei 15°C/60 bar Wasserstoffdruck nach 13 min Reaktionszeit bei 61 %igem Umsatz 81 % Butylenoxid, bezogen auf umgesetztes Vinyloxiran, erhalten. Mit Methanol als Lösungsmittel wird dagegen unter gleichen Reaktionsbedingungen bei einem Umsatz von 86 % eine Butylenoxid-Selektivität von nur 53 % erhalten, wobei 13 % Butanol und 18 % Butenolen gebildet werden. Nachteilig an diesem Verfahren ist, daß eine hohe Selektivität für die Bildung von 1,2-Butylenoxid nur bei einem relativ geringen Teilumsatz des Vinyloxirans erzielt werden. Da Vinyloxiran und 1,2-Butylenoxid destillativ praktisch nicht voneinander getrennt werden können, kommt diesem Verfahren somit keine technische Bedeutung zu. Mit Palladium-Katalysatoren auf Polymerbasis werden bei einem Umsatz von 68 % maximale Butylenoxid-Selektivitäten von 60 % erreicht, wobei Butenole bzw. n-Butanol in einer Selektivität von 18 % bzw. 4 % gebildet werden.

Mit Kupfer-haltigen Katalysatoren wird eine niedrigere Hydrieraktivität und Verharzung des Hydrieraustrages beobachtet, die diese Verfahren technisch inpraktikabel macht. Bei Reaktionstemperaturen von 60-100°C, 60 bar H₂ und 30 Gew.-% Katalysator wird nach 3 h Reaktionszeit ein Vinyloxiran-Umsatz von 50 % und eine Butylenoxid-Selektivität von 70 % erzielt.

Die Deutsche Patentanmeldung P 44 22 046.4 betrifft durch Tränkung hergestellte Katalysatoren zur Verwendung in der selektiven Hydrierung von Vinyloxiran zu 1,2-Butylenoxid. Trotz der hohen Selektivitäten, die darin beschrieben werden, entstehen als Nebenprodukt relativ große Mengen an Butyraldehyd.

Die Deutsche Patentanmeldung P 44 07 486.7 lehrt die Hydrierung von Vinyloxiran an Katalysatoren, die durch Aufdampfen der katalytisch aktiven Elemente auf einen Träger aus Metallfolie bzw. Metalldrahtgewebe erhalten werden. Diese Katalysatoren ermöglichen eine hochselektive Umsetzung zum gewünschten Verfahrensprodukt, jedoch sind die eingesetzten Träger relativ teuer.

Der vorliegenden Erfindung lag somit die Aufgabe zugrunde, ein wirtschaftliches Verfahren zur Herstellung von 1,2-Butylenoxid aus Vinyloxiran zu finden, bei dem 1,2-Butylenoxid in hoher Ausbeute und Selektivität gebildet wird. Weiterhin war es Ziel, für diesen Zweck Katalysatoren zu finden, die diese Aufgabe erfüllen und im Vergleich zu den Katalysatoren des Standes der Technik wesentlich geringere Mengen an teuren Edelmetallen als Katalysatorkomponente benötigen. Schließlich sollte ein Verfahren gefunden werden, in dem Katalysatoren eingesetzt werden, die aus preiswerten Trägermaterialien hergestellt werden können.

Dementsprechend wurde ein Verfahren zur Herstellung von 1,2-Butylenoxid durch katalytische Hydrierung von Vinyloxiran an einem Heterogenkatalysator gefunden, das dadurch gekennzeichnet ist, daß man einen Katalysator verwendet, der durch Abscheidung eines oder mehrerer katalytisch aktiver Elemente der Gruppen 7 bis 11 des Periodischen Systems der Elemente aus der Gasphase auf einem inerten, nichtmetallischen Träger hergestellt wird.

Das erfindungsgemäße Verfahren ermöglicht es überraschenderweise, die Doppelbindung des Vinyloxirans gemäß Gleichung (1) selektiv zu hydrieren, 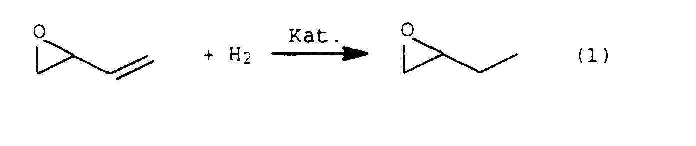 ohne daß der empfindliche Epoxidring bei der Hydrierung in nennenswertem Umfang hydrogenolytisch gespalten wird und ohne daß es in nennenswertem Umfang zu anderen Nebenreaktionen, z.B. zur Isomerisierung des Vinyloxirans, beispielsweise zu Crotonaldehyd, das in Folge zu Crotylalkohol und Butanol hydriert wird.

Die erfindungsgemäß verwendeten Katalysatoren können hergestellt werden, indem man eines oder mehrere Elemente der Gruppen 7 bis 11 des Periodischen Systems der Elemente, die früher auch als I., VII. und VIII. Nebengruppe bezeichnet wurden, insbesondere Kupfer, Rhenium, Ruthenium, Kobalt, Nickel, Palladium oder Platin oder Gemische dieser Elemente beispielsweise durch physikalische Gasphasenabscheidung (PVD) und/oder chemische Gasphasenabscheidung (CVD) auf einem Träger abscheidet. Katalysatoren mit Palladium, Cobalt, Nickel, sowie solche mit Kupfer und Nickel als aktive Elemente sind besonders bevorzugt.

PVD- bzw. CVD-Verfahren sind beispielsweise beschrieben in R.F. Bhunshah et al, "Deposition Technologies for Films and Coatings", Noyes Publications, 1982. Geeignete PVD-Verfahren sind z.B. das Aufdampfen, die Kathodenzerstäubung (Sputtern) oder die Lichtbogenbeschichtung, bevorzugt die Kathodenzerstäubung. Bekannte CVD-Verfahren sind die thermische CVD und die plasmaunterstützte CVD.

Beim Aufdampfen wird das Beschichtungsmaterial, d.h. eines oder mehrere Elemente der Gruppen 7 bis 11 des Periodischen Systems der Elemente, in an sich bekannter Weise in eine geeignete Aufdampfquelle wie elektrisch geheizte Verdampferschiffchen oder Elektronenstrahlverdampfer eingefüllt. Das Beschichtungsmaterial, in der Regel ein Metall oder eine Legierung, wird danach unter vermindertem Druck, üblicherweise im Bereich von 10⁻⁷ bis 10⁻³ mbar, erhitzt, wobei ein Teil des Beschichtungsmaterials verdampft und sich auf dem Substrat, d.h. entsprechend der Erfindung auf inerten, nichtmetallischen Trägern, als Schicht niederschlägt. Die Aufdampfrate der Schicht kann über die Temperatur der Aufdampfquelle gesteuert werden.

Bei der Kathodenzerstäubung wird das Beschichtungsmaterial in fester Form als sogenanntes Target auf die Kathode eines Plasmasystems aufgebraucht, im Vakuum (vorzugsweise 5 x 10⁻⁴ bis 1 x 10⁻¹ mbar) in einer Prozeßgasatmosphäre durch Anlegen eines Plasmas zerstäubt und auf dem zu beschichtenden Träger abgeschieden. Üblicherweise enthält das Prozeßgas ein Edelgas wie Argon.

Bei der Lichtbogenbeschichtung erfolgt der Abtrag des Beschichtungsmaterials mit einem elektrischen Lichtbogen, der zu einem hohen Ionisationsgrad des Beschichtungsmaterials in der Prozeßgasatmosphäre führt. Der zu beschichtende Träger kann mit einer in der Regel negativen Vorspannung versehen werden, welche zu einem intensiven Ionenbeschuß während der Beschichtung führt.

Bei der CVD-Abscheidung der erfindungsgemäßen Schichten wird ein Gasgemisch mit mindestens einer metallorganischen Ausgangsverbindung ausreichender Flüchtigkeit eines Elements der Gruppen 7 bis 11 des Periodensystems in den Beschichtungsraum eingeführt und durch thermische Energiezufuhr (thermische CVD) oder unter Einwirkung eines Plasmas (plasmaunterstützte CVD) zersetzt, wobei sich auf dem Träger die gewünschte Schicht bildet. Das eingesetzte Gasgemisch kann darüber hinaus Inertgase wie He, Ne, Ar, Kr oder Xe und weitere Reaktivgase enthalten. Die Abscheidung erfolgt in einem Druckbereich zwischen 10⁻⁴ und 10⁺³ mbar. Als Ausgangsverbindungen kommen z.B. Carbonylverbindungen, Acetylacetonate und Cyclopentadienylverbindungen der katalytisch aktiven Elemente in Betracht.

Zur Herstellung der erfindungsgemäßen Schichten mit der bevorzugten Methode der Kathodenzerstäubung sind verschiedene methodische Varianten wie Magnetron-Sputtern, DC- bzw. RF-Sputtern, Bias-Sputtern oder reaktives Sputtern sowie deren Kombinationen geeignet. Beim Magnetron-Sputtern befindet sich das zu zerstäubende Target in einem äußeren Magnetfeld, welches das Plasma in den Bereich des Targets konzentriert und damit eine Erhöhung der Zerstäubungsrate bewirkt. Beim DC- bzw. RF-Sputtern erfolgt die Anregung des Zerstäubungsplasmas durch eine Gleichspannung (DC) oder durch eine Wechselspannung (RF). Beim Bias-Sputtern wird das zu beschichtende Substrat mit einer in der Regel negativen Vorspannung (Bias) belegt, welche während der Beschichtung zu einem intensiven Beschuß des Substrats mit Ionen führt.

Die Einstellung der Schichtdicke, der chemischen Zusammensetzung und der Mikrostruktur der Schichten erfolgt wie nachfolgend beschrieben durch die Beschichtungsparameter Prozeßgasdruck, Zerstäubungsleistung, Sputtermodus, Substrattemperatur und Beschichtungszeit.

Durch die Wahl entsprechender Sputterleistungen und Beschichtungszeiten kann die Dicke der Sputterschicht bequem zwischen wenigen Atomlagen und ca. 10 µm gewählt werden. Für das erfindungsgemäße Verfahren sind Schichtdicken zwischen 1 und 1000 nm bevorzugt.

Die Herstellung mehrkomponentiger Aktivschichten kann durch Zerstäuben eines geeigneten mehrkomponentigen Targets erfolgen. Geeignete Targets sind entweder homogene Legierungstargets, die in bekannter Weise durch Schmelzverfahren bzw. durch pulvermetallurgische Methoden hergestellt werden, oder inhomogene Mosaiktargets, die durch Zusammenfügen kleinerer Teilstücke unterschiedlicher chemischer Zusammensetzung oder durch Auflegen bzw. Aufkleben von kleinen scheibenförmigen Materialstücken auf homogene Targets hergestellt werden. In alternativer Weise können metallische Legierungen dadurch hergestellt werden, daß zwei oder mehrere Targets unterschiedlicher Zusammensetzung gleichzeitig zerstäubt werden (simultanes Sputtern).

Mit den genannten Abscheideverfahren ist es auch denkbar, dünne Gradientenschichten oder Vielfachschichten herzustellen, deren Zusammensetzung mit zunehmender Schichttiefe mit den genannten Prozeßparametern in definierter Weise variiert wird.

Die Mikrostruktur (z.B. Phasenverteilung, Kristallitform und -größe, kristallographische Ausrichtung, Porosität) der Aktivschichten kann ebenfalls durch die genannten Prozeßparameter in weiten Grenzen gesteuert werden. So führt beispielsweise die Magnetron-Zerstäubung eines Metall-Targets im Druckbereich zwischen 4 x 10⁻³ und 8 x 10⁻³ mbar bei Schichtdicken zwischen 20 und 500 nm zu relativ dichten und porenfreien Schichten, während oberhalb 10⁻² mbar eine säulenförmige Morphologie mit zunehmender Porosität auftritt. Bei Schichtdicken unter ca. 50 nm erfolgt - abhängig von der Rauhigkeit des Trägers - in der Regel ein Inselwachstum der Schichten. Neben dem Sputterdruck und dem Träger beeinflußt die Substrattemperatur sowie der Ionenbeschuß während der Beschichtung die Mikrostruktur der Schichten. Für das erfindungsgemäße Verfahren ist im Falle von Pd-Aktivschichten (20 nm) beispielsweise ein Sputterdruck zwischen 1 und 10 x 10⁻² mbar bevorzugt.

Um eine gleichmäßige Beschichtung der Träger zu erzielen, ist es notwendig, die Trägermaterialien während der Beschichtung mit geeigneten mechanischen oder strömungsmechanischen Vorrichtungen in Bewegung zu halten. Geeignete mechanische Vorrichtungen hierfür sind beispielsweise periodisch bewegte Käfige, Trommeln, Schalen oder Rinnen, in welchen die zu beschichtenden Träger zu statistischen Bewegungen angeregt werden. Alternativ ist denkbar, daß die zu beschichtenden Träger durch ein Wirbelbettverfahren in statistischer Bewegung gehalten werden (vgl. EP-A 655 516).

Als Träger für die im erfindungsgemäßen Verfahren anwendbaren Katalysatoren kommen beispielsweise Formkörper aus Glas, Quarzglas, Keramik, Titandioxid, Zirkondioxid, Aluminiumoxid, Alumosilikate, Borate, Steatit, Magnesiumsilikat, Siliciumdioxid, Silicate, Kohlenstoff, z.B. Graphit, oder Mischungen der genannten Materialien in Frage. Bevorzugt werden Steatit, Siliciumdioxid und Aluminiumoxid. Der Träger kann porös oder nicht porös sein. Als Formkörper kommen Stränge, Tabletten, Wagenräder, Sterne, Monolithe, Kugeln, Splitt, Ringe oder Extrudate in Betracht. Besonders bevorzugt sind Kugeln, Tabletten und Stränge. Die Wahl der Formkörper ist nicht primär durch die Herstellweise der erfindungsgemäße zu verwendenden Katalysatoren beschränkt, sondern durch die Art der Verwendung des gewünschten Katalysators, beispielsweise als Suspensions- oder Festbettkatalysator. So können Kugeln z.B. 100 µm bis 2 mm groß sein, Stränge 1 bis 5 mm dick und Splitt 0,1 bis 10 mm groß sein.

Durch die beschriebenen Techniken zur Abscheidung der katalytisch aktiven Elemente können gleichzeitig oder nacheinander auch Promotoren auf die Träger aufgebracht werden. Als Promotoren kommen vor allem Elemente der Gruppe 4 des Periodischen Systems der Elemente (früher: IV. Nebengruppe) in Betracht, insbesondere Zirkonium.

Die so hergestellten Katalysatoren können direkt im erfindungsgemäßen Verfahren verwendet werden, vorteilhaft werden sie vor ihrem Einsatz im erfindungsgemäßen Verfahren aber reduziert, und zwar im allgemeinen mit Wasserstoff oder Wasserstoff-haltigen Gasen bei Temperaturen von typischerweise 50 bis 300°C, vorzugsweise von 80 bis 250°C. Die Reduktion wird im allgemeinen solange durchgeführt, bis kein Wasser mehr gebildet wird. Durch diese Reaktion können durch Sauerstoffspuren während der Abscheidung oder durch Reaktion der katalytisch aktiven Elemente an Luft gebildete Oxid- oder Adsorbatschichten beseitigt werden. Wasserstoff kann mit inerten Gasen wie CO₂, Argon oder Stickstoff verdünnt eingesetzt werden.

Zur Durchführung des erfindungsgemäßen Verfahrens wird Vinyloxiran oder Lösungen des Vinyloxirans in einem unter den Reaktionsbedingungen inerten Lösungsmittel in Gegenwart der erfindungsgemäß zu verwendenden Katalysatoren bei Temperaturen von im allgemeinen 0 bis 200°C, vorzugsweise von 10 bis 130°C, insbesondere von 20 bis 100°C und besonders bevorzugt bei 25 bis 60°C bei einem Druck von im allgemeinen 1 bis 300 bar, vorzugsweise von 1 bis 100 bar und besonders bevorzugt von 1 bis 50 bar hydriert.

Das erfindungsgemäße Verfahren kann ohne Lösungsmittel oder vorteilhaft in Anwesenheit eines unter den Reaktionsbedingungen inerten Lösungsmittels durchgeführt werden. Derartige Lösungsmittel können beispielsweise sein: Ether wie Tetrahydrofuran, Dioxan, Methyl-tert.-butylether, Di-n-butylether, Dimethoxyethan oder Diisopropylether, Alkohole wie Methanol, Ethanol, Propanol, Isopropanol, n-Butanol, Isobutanol oder tert.-Butanol, C₂- bis C₄-Glycole, Kohlenwasserstoffe wie Petrolether, Benzol, Toluol oder Xylol, N-Alkyl-Lactame, wie N-Methylpyrrolidon oder N-Octylpyrrolidon.

Das erfindungsgemäße Verfahren kann sowohl kontinuierlich als auch diskontinuierlich ausgeübt werden. Bei der kontinuierlichen Betriebsweise können beispielsweise Rohrreaktoren vorteilhaft eingesetzt werden, in denen der Katalysator als Festbett angeordnet ist, über das die Reaktionsmischung in Sumpf- oder Rieselfahrweise geleitet werden kann. Bei der diskontinuierlichen Betriebsweise können sowohl einfache Rührreaktoren oder auch vorteilhaft Schlaufenreaktoren verwendet werden.

Die Aufarbeitung der Reaktionsmischung zur Isolierung des 1,2-Butylenoxids kann auf herkömmliche Weise, z.B. durch Destillation, erfolgen.

Das als Ausgangsmaterial benötigte Vinyloxiran kann z.B. nach dem Verfahren von US-A 4,897,498 durch partielle Oxidation von 1,3-Butadien an Silberkatalysatoren hergestellt werden.

1,2-Butylenoxid findet z.B. als Kraftstoffadditiv oder als Stabilisator von Chlorkohlenwasserstoffen Verwendung.

### Beispiele

### Beispiel 1

### Herstellung von Katalysatoren durch Kathodenzerstäubung

### a) Herstellung von erfindungsgemäßen Katalysatoren

Als Kathodenzerstäubungsanlage wurde eine Sputteranlage Alcatel SCM 850 verwendet. Verschiedene Träger in Kugelform (vgl. Tabelle 1) wurden auf ein rundes Stahlnetz (Durchmesser 150 mm) mit einer Maschenweite von ca. 1 mm aufgelegt und in eine Kathodenzerstäubungsanlage eingebracht. In einem Abstand von 70 mm wurde ein Target entsprechend Tabelle 1 eingesetzt. Die Anlage wurde evakuiert. Danach wurde Argon bis zu einem Druck entsprechend Tabelle 1 eingelassen. Durch Anlegen einer geeigneten Spannung an das Target wurde auf die Träger eine Schicht abgei schieden. Die Träger wurden dabei durch eine mechanische Anregung des Stahlnetzes statistisch bewegt, um eine homogene Beschichtung zu gewährleisten. Die Ergebnisse sind in Tabelle 1 zusammengestellt.

**Tabelle 1**

| Kat. Nr. | Trägermaterial | Kugelgröße | Target | Leistung [W] | Sputterdruck [mbar] | Schichtdicke [nm] |
|---|---|---|---|---|---|---|
| 1 | Steatit | 2 mm | Pd | 250 (RF) | 5 x 10⁻² | 20 |
| 2 | Steatit | 2 mm | Pd | 250 (RF) | 1 x 10⁻² | 20 |
| 3 | Steatit | 2 mm | Pd | 250 (RF) | 5 x 10⁻³ | 1 |
| 4 | Steatit | 2 mm | Pd | 250 (RF) | 5 x 10⁻³ | 10 |
| 5 | Steatit | 2 mm | Pd | 500 (RF) | 5 x 10⁻³ | 100 |
| 6 | Steatit | 2 mm | Pd | 500 (RF) | 5 x 10⁻³ | 1000 |
| 7 | SiO₂ | 1,5-3,5 mm | Pd | 250 (RF) | 5 x 10⁻³ | 20 |
| 8 | Al₂O₃ | 1,4-2,8 mm | Pd | 250 (RF) | 5 x 10⁻³ | 20 |
| 9 | Glas | 2 mm | Pd | 500 (RF) | 5 x 10⁻² | 1000 |
| 5 10 | Steatit | 2 mm | Ni₃₀Cu₇₀* | 250 (DC) | 5 x 10⁻² | 10 |
| 11 | Steatit | 2 mm | Ni₂₀Cu₈₀* | 250 (DC) | 5 x 10⁻² | 20 |
| 12 | Steatit | 2 mm | Ni₃₀Cu₇₀* | 250 (DC) | 5 x 10⁻² | 100 |
| 13 | Steatit | 2 mm | Ni | 1000 (RF) | 5 x 10⁻² | 5 |
| 14 | Steatit | 2 mm | Co | 250 (DC) | 5 x 10⁻² | 10 |
| 15 | Steatit | 2 mm | Cu | 500 (DC) | 5 x 10⁻² | 10 |
| 16 | Steatit | 2 mm | Re | 500 (DC) | 5 x 10⁻² | 10 |
| 17 | Steatit | 2 mm | Ru | 500 (DC) | 5 x 10⁻² | 100 |
| RF = Wechselspannung; DC = Gleichspannung; * Mosaiktarget | | | | | | |

### b) Herstellung von Pd/Zr- und Pt/Zr-haltigen Katalysatoren

Die in Tabelle 2 aufgeführten Katalysatoren wurden durch Kathodenzerstäubung von amorphen Legierungen hergestellt, wobei für die Katalysatoren Nr. 18 und 19 ein Target (Pd₁Zr₂) und für Katalysator Nr. 20 zwei Targets (Pt, Zr) verwendet wurden.

Die Katalysatoren Nr. 18 und 19 wurden anschließend bei 280°C für 24 h mit einer Wasserstoff/Kohlendioxid-Mischung nachbehandelt (14 l/h H₂, 4 l/h CO₂).

Die Nachbehandlung von Katalysator Nr. 20 erfolgte bei 320°C für 24 h mit einer Wasserdampf/Stickstoff-Mischung (40 l/h N₂, 3 g/l H₂O).

**Tabelle 2**

| Kat. Nr. | Trägermaterial | Kugelgröße | Target 1 | Leistung 1 [W] | Target 2 | Leistung 2 [W] | Sputterdruck [mbar] | Schichtdicke [nm] |
|---|---|---|---|---|---|---|---|---|
| 18 | Steatit | 2 mm | Pd₁Zr₂ | 500(RF) - | - | - | 2,5x10⁻² | 1000 |
| 19 | Glas | 2 mm | Pd₁Zr₂ | 500(RF) - | - | - | 5x10⁻² | 300 |
| 20 | Steatit | 2 mm | Pt | 350(RF) | Zr | 1000 (DC) | 5x10⁻³ | 1000 |

### Beispiel 2

In einem 50 ml fassenden Autoklaven wurde die zu hydrierende Lösung aus 2,5 g Vinyloxiran und 22,5 g Tetrahydrofuran mit 0,5 g Katalysator Nr. 1 ohne vorherige Aktivierung mit Wasserstoff versetzt und unter Rühren 8 h bei 25°C und 40 bar mit Wasserstoff hydriert. Bei einem Umsatz von 100 % wurden 91,7 Mol-% 1,2-Butylenoxid, 1,0 Mol-% n-Butyraldehyd und 2,6 Mol-% n-Butanol erhalten.

### Beispiele 3 - 22

2,5 g Vinyloxiran in 22,5 g Tetrahydrofuran wurden analog zu Beispiel 2 an den Katalysatoren 2-20 bei 40 bar mit H₂ hydriert. Tabelle 3 zeigt die Reaktionsbedingungen und Zusammensetzungen der Hydrierausträge. Die Angabe Mol-% bezieht sich auf umgesetztes Vinyloxiran. Wurden die Katalysatoren vor Gebrauch aktiviert, so erfolgte dies bei 250°C in einer Wasserstoffatmosphäre.

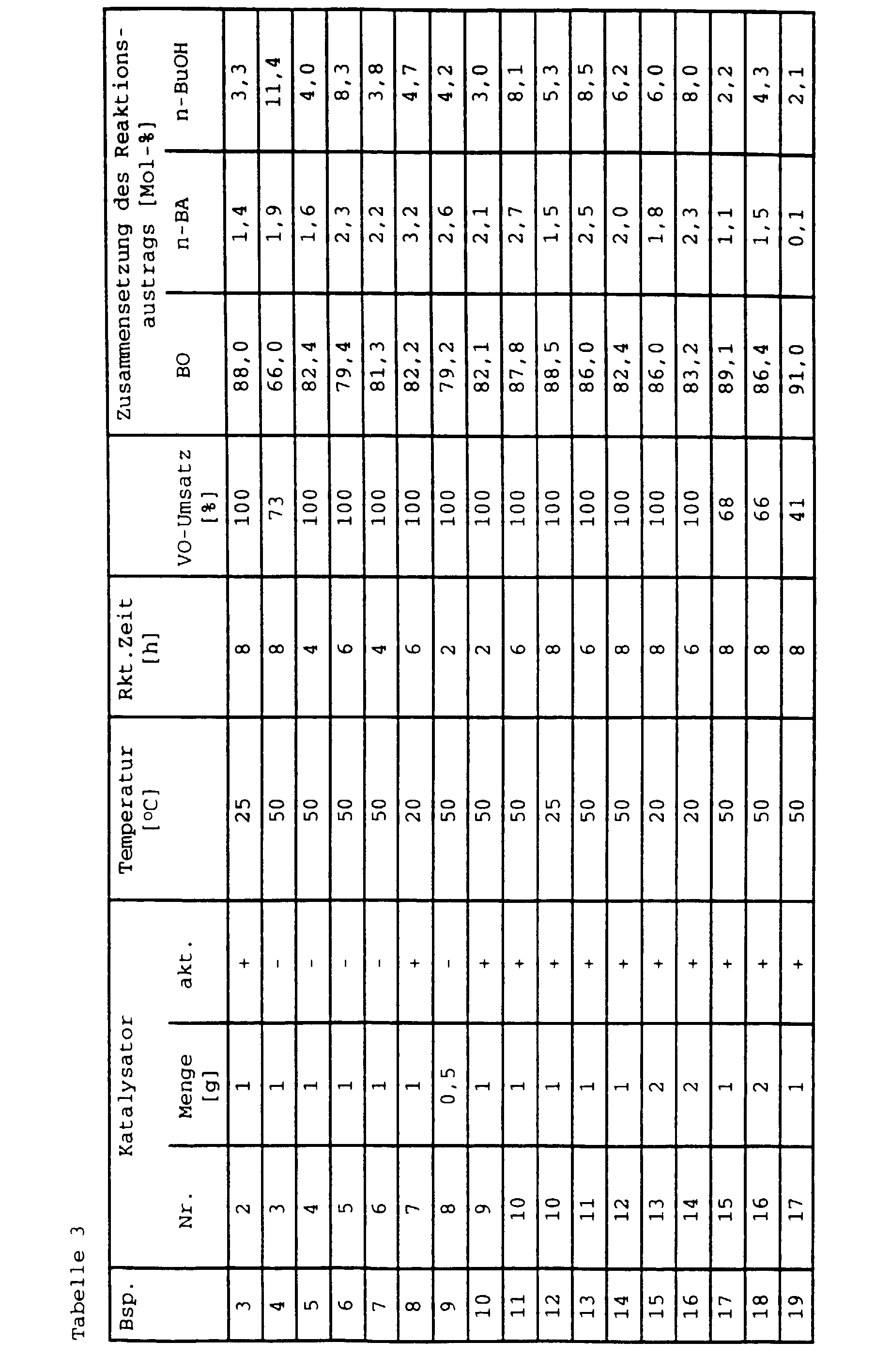

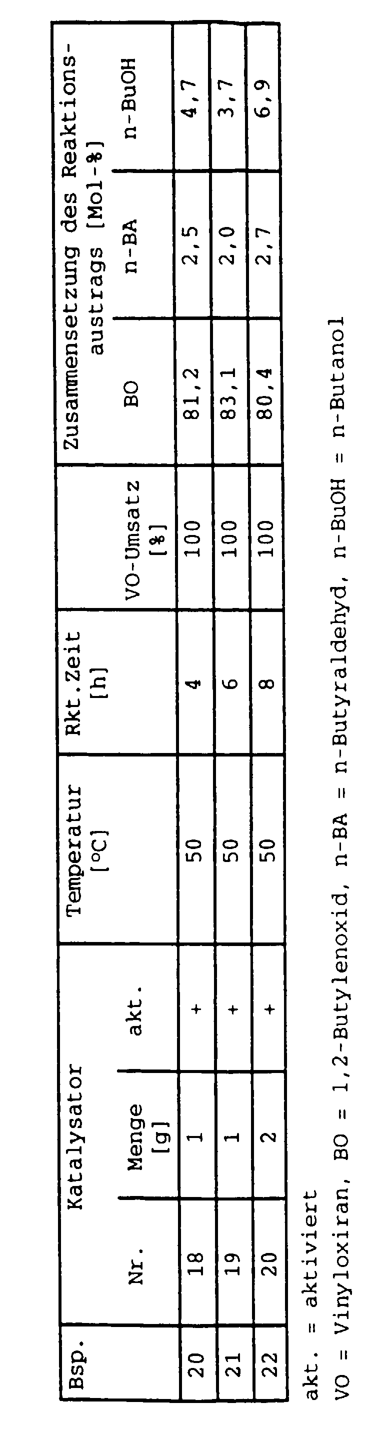

## Patentansprüche

1. Verfahren zur Herstellung von 1,2-Butylenoxid durch katalytische Hydrierung von Vinyloxiran an einem Heterogenkatalysator, dadurch gekennzeichnet, daß man einen Katalysator verwendet, der durch Abscheidung eines oder mehrerer katalytisch aktiver Elemente der Gruppen 7 bis 11 des Periodischen Systems der Elemente aus der Gasphase auf einem inerten, nichtmetallischen Träger hergestellt wird.

2. Verfahren nach Anspruch 1, dadurch gekennzeichnet, daß man Palladium, Cobalt, Nickel oder eine Mischung aus Kupfer und Nickel als katalytisch aktive Elemente verwendet.

3. Verfahren nach Anspruch 1 oder 2, dadurch gekennzeichnet, daß man Steatit, Siliciumdioxid oder Aluminiumoxid als inerten Träger verwendet.

4. Verfahren nach den Ansprüchen 1 bis 3, dadurch gekennzeichnet, daß man die katalytisch aktiven Elemente durch Kathodenzerstäubung in die Gasphase überführt.

5. Verfahren nach den Ansprüchen 1 bis 4, dadurch gekennzeichnet, daß man Katalysatoren mit einer Schichtdicke der katalytisch aktiven Elemente von 1 bis 1000 nm verwendet.

6. Verfahren nach den Ansprüchen 1 bis 5, dadurch gekennzeichnet, daß man die Katalysatoren vor ihrer Verwendung bei 50 bis 300°C mit Wasserstoff behandelt.

7. Verfahren nach den Ansprüchen 1 bis 6, dadurch gekennzeichnet, daß neben den katalytisch aktiven Elementen Promotoren aus der Gruppe 4 des Periodischen Systems der Elemente aus der Gasphase auf dem Träger abgeschieden werden.

## Claims

1. A process for preparing 1,2-butylene oxide by catalytic hydrogenation of vinyloxirane over a heterogeneous catalyst, wherein the catalyst used is produced by deposition of one or more catalytically active elements of groups 7 to 11 of the Periodic Table of the Elements from the gas phase onto an inert, nonmetallic support.

2. A process as claimed in claim 1, wherein the catalytically active elements used are palladium, cobalt, nickel or a mixture of copper and nickel.

3. A process as claimed in claim 1 or 2, wherein the inert support used is steatite, silicon dioxide or aluminum oxide.

4. A process as claimed in any of claims 1 to 3, wherein the ctalytically active elements are brought into the gas phase by cathode atomization.

5. A process as claimed in any of claims 1 to 4, wherein catlaysts having a layer thickness of the catalytically active elements of from 1 to 1000 nm are used.

6. A process as claimed in any of claims 1 to 5, wherein the catalysts are treated at from 50 to 300°C with hydrogen prior to use.

7. A process as claimed in any of claims 1 to 6, wherein not only the catalytically active elements but also promoters from group 4 of the Periodic Table of the Elements are deposited from the gas phase onto the support.

## Revendications

1. Procédé de préparation de l'oxyde de 1,2-butylène par hydrogénation catalytique du vinyloxiranne sur un catalyseur hétérogène, caractérisé en ce que l'on emploie un catalyseur qui est préparé par dépôt d'un ou plusieurs éléments à effet catalytique des Groupes 7 à 11 du Tableau Périodique des Eléments, à partir de la phase gazeuse sur un support inerte non métallique.

2. Procédé selon la revendication 1, caractérisé en ce que l'on emploie le palladium, le cobalt, le nickel ou un mélange de cuivre et de nickel en tant qu'éléments à effet catalytique.

3. Procédé selon la revendication 1 ou 2, caractérisé en ce que l'on emploie la stéatite, le dioxyde de silicium ou l'oxyde d'aluminium en tant que support inerte.

4. Procédé selon les revendications 1 à 3, caractérisé en ce que l'on met les éléments à effet catalytique en phase gazeuse par pulvérisation cathodique.

5. Procédé selon les revendications 1 à 4, caractérisé en ce que l'on emploie des catalyseurs ayant une épaisseur de couche d'éléments à effet catalytique de 1 à 1 000 nm.

6. Procédé selon les revendications 1 à 5, caractérisé en ce que l'on traite les catalyseurs avec de l'hydrogène à une température de 50°C à 300°C avant leur emploi.

7. Procédé selon les revendications 1 à 6, caractérisé en ce que, en plus des éléments à effet catalytique, des activateurs issus du Groupe 4 du Tableau Périodique des Eléments sont déposés à partir de la phaze gazeuse sur le support.
